(19) Europäisches Patentamt / European Patent Office / Office européen des brevets

(11) **EP 4 289 806 A1**

(12) **EUROPEAN PATENT APPLICATION**
published in accordance with Art. 153(4) EPC

(43) Date of publication:
**13.12.2023 Bulletin 2023/50**

(21) Application number: **22749569.4**

(22) Date of filing: **26.01.2022**

(51) International Patent Classification (IPC):
***C07C 1/20*** (2006.01)        ***C07C 11/06*** (2006.01)

(52) Cooperative Patent Classification (CPC):
(C-Sets available)
**C07C 1/20; B01J 21/066; C07C 1/24; C07C 11/06;**
C07C 2521/06; C07C 2523/02        (Cont.)

(86) International application number:
**PCT/JP2022/002835**

(87) International publication number:
**WO 2022/168695 (11.08.2022 Gazette 2022/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **04.02.2021   JP 2021016728**

(71) Applicant: **Sumitomo Chemical Company Limited
Tokyo 103-6020 (JP)**

(72) Inventors:
• **SUZUKI, Tetsuo**
  **Niihama-shi, Ehime 792-8521 (JP)**
• **YAMASHITA, Fumikazu**
  **Ichihara-shi, Chiba 299-0195 (JP)**
• **HASHIMOTO, Aika**
  **Ichihara-shi, Chiba 299-0195 (JP)**

(74) Representative: **Winter, Brandl - Partnerschaft
mbB
Alois-Steinecker-Straße 22
85354 Freising (DE)**

(54)    **METHOD FOR PRODUCING PROPYLENE**

(57)    A propylene production method according to an aspect of the present invention includes: a reaction step of supplying a fresh raw material and a recycled raw material to a reactor to obtain a crude product; a gas-liquid separation step of separating the crude product into a gas phase and a liquid phase; a propylene recovery step of recovering propylene from the gas phase; and a recycling step of recovering the recycled raw material from the liquid phase.

FIG. 1

(52) Cooperative Patent Classification (CPC): (Cont.)

C-Sets
**C07C 1/20, C07C 11/06;**
**C07C 1/24, C07C 11/04**

**Description**

Technical Field

The present invention relates to a propylene production method.

Background Art

Non-patent Literature 1 discloses a method for producing propylene using ethanol as a raw material. Citation List

[Non-patent Literature]

[Non-patent Literature 1]
Iwamoto, M. et al., Pulse and IR study on the reaction pathways for the conversion of ethanol to propene over scandium-loaded indium oxide catalysts, ACS Catal., 4(10), 3463-3469, 2014

Summary of Invention

Technical Problem

However, a conventional technique as described above has a problem that a carbon-based yield of propylene is low.
An object of an aspect of the present invention is to provide a propylene production method that makes it possible to increase a carbon-based yield of propylene.

Solution to Problem

In order to attain the object, a propylene production method according to an aspect of the present invention includes: a reaction step of supplying a fresh raw material containing ethanol and acetone and a recycled raw material containing acetone to a reactor so that the fresh raw material and the recycled raw material react to obtain a crude product; a gas-liquid separation step of separating the crude product into a gas phase containing propylene and a liquid phase containing acetone; a propylene recovery step of recovering propylene from the gas phase; and a recycling step of recovering the recycled raw material from the liquid phase.

Advantageous Effects of Invention

According to an aspect of the present invention, it is possible to increase a carbon-based yield of propylene.

Brief Description of Drawings

Fig. 1 is a schematic diagram illustrating a process of a propylene production method according to an aspect of the present invention.
Fig. 2 is a process flowchart used in process simulation in Example 1.

Description of Embodiments

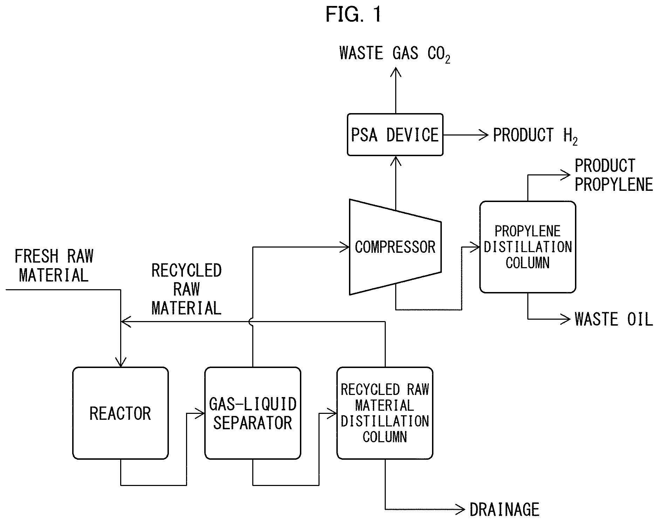

The following description will discuss details of a propylene production method according to an aspect of the present invention, with reference to Fig. 1. Fig. 1 is a schematic diagram illustrating an example of a device that is used in the propylene production method according to an aspect of the present invention. The propylene production method according to an aspect of the present invention includes: a reaction step of supplying a fresh raw material containing ethanol and acetone and a recycled raw material containing acetone to a reactor so that the fresh raw material and the recycled raw material react to obtain a crude product; a gas-liquid separation step of separating the crude product into a gas phase containing propylene and a liquid phase containing acetone; a propylene recovery step of recovering propylene from the gas phase; and a recycling step of recovering the recycled raw material from the liquid phase. The recycled raw material recovered in the recycling step is supplied to the reaction step.

(Reaction step)

The reaction step is a step of supplying a fresh raw material containing ethanol and acetone and a recycled raw material containing acetone to a reactor so that the fresh raw material and the recycled raw material react to obtain a crude product. The reaction step is carried out, for example, in a reactor illustrated in Fig. 1, where a fresh raw material and a recycled raw material are supplied to the reactor.

In this specification, the term "fresh raw material" is intended to be a raw material that is newly supplied to the reactor from outside of the reaction system. The fresh raw material may be purchased or recovered from a reaction system other than the present reaction system. The fresh raw material may be a biomass raw material. The term "recycled raw material" described below is intended to be a raw material that contains at least acetone and that is recovered from a liquid phase separated in the gas-liquid separation step. The term "raw material" is intended to be a mixture of a fresh raw material and other components (including a recycled raw material) supplied to the reactor. Examples of ethanol include, but not particularly limited to, biomass-derived ethanol, ethanol produced using carbon oxide and hydrogen as raw materials, and ethanol produced using, as a raw material, waste gas that contains hydrogen and carbon dioxide and that is separated in the propylene recovery step described later. These kinds of ethanol can be used alone or in combination of two or more thereof.

Examples of carbon oxide used as a raw material of ethanol include: carbon oxide produced by decomposing biomass; carbon oxide produced from a fossil raw material; carbon oxide generated in the process of using a fossil resource as energy; carbon oxide generated in the process of producing iron and steel or a chemical product; carbon oxide produced using plastic as a raw material; and carbon dioxide recovered from air. These kinds of carbon oxide can be used alone or in combination of two or more thereof. Hydrogen used as a raw material of ethanol may be, for example, hydrogen derived from a fossil raw material and/or hydrogen produced by electrolysis of water. Examples of acetone include, but not particularly limited to, acetone obtained by fermentation and/or acetone produced using aromatic peroxide as a raw material. The fresh raw material and the recycled raw material may contain, in addition to ethanol and acetone, at least one selected from the group consisting of oxygenated hydrocarbons such as isopropanol and acetaldehyde, and water. If the fresh raw material contains oxygenated hydrocarbons such as isopropanol and acetaldehyde, the oxygenated hydrocarbons may be, but not particularly limited to, oxygenated hydrocarbons such as isopropanol and acetaldehyde obtained by fermentation, or oxygenated hydrocarbons such as isopropanol and acetaldehyde derived from a fossil raw material.

In the reaction step, a molar ratio of acetone contained in a fresh raw material supplied to the reactor to ethanol contained in the fresh raw material is preferably not less than 0.010, more preferably not less than 0.25, and further preferably not less than 0.30. The molar ratio is preferably not more than 1.70, more preferably not more than 1.60, and further preferably not more than 1.50. If the molar ratio falls within such ranges, it is possible to further increase a carbon-based yield of propylene. Here, the carbon-based yield of propylene is a proportion of the number of moles of carbon contained in propylene recovered in the propylene recovery step to a total number of moles of carbon contained in oxygenated hydrocarbons (such as ethanol, acetone, isopropanol, and acetaldehyde) contained in the fresh raw material supplied to the reactor.

A molar ratio of a total amount of acetone to a total amount of ethanol which are contained in the fresh raw material and the recycled raw material supplied to the reactor is preferably not less than 0.010, more preferably not less than 0.25, and further preferably not less than 0.30. The molar ratio is preferably not more than 1.7, more preferably not more than 1.60, and further preferably not more than 1.50.

The crude product obtained in the reaction step contains at least propylene and acetone, and may further contain ethanol and/or isopropanol. The crude product obtained in the reaction step may contain a low boiling point component (e.g., acetaldehyde and the like) having a boiling point lower than that of acetone.

From the viewpoint of increasing a carbon-based yield of propylene, a carbon-based propylene concentration in the crude product at the reactor outlet is preferably not less than 5%, more preferably not less than 10%, and further preferably not less than 15%. Here, the carbon-based propylene concentration is a proportion of the number of moles of carbon contained in propylene at the reactor outlet to a total number of moles of carbon contained in a produced gas, at the reactor outlet, that is obtained by removing, from the crude product, an inert component supplied to the reactor.

From the viewpoint of increasing a carbon-based yield of propylene, a carbon-based acetone concentration in the crude product at the reactor outlet is preferably not less than 1%, more preferably not less than 5%, and further preferably not less than 10%. Here, the carbon-based acetone concentration is a proportion of the number of moles of carbon contained in acetone at the reactor outlet to a total number of moles of carbon contained in a produced gas, at the reactor outlet, that is obtained by removing, from the crude product, an inert component supplied to the reactor.

In the reaction step, it is preferable to also supply water to the reactor. In the reaction step, ethanol may cause a dehydration reaction to generate ethylene as a by-product. However, by supplying water to the reactor, such generation of ethylene as a by-product can be reduced. As a result, it is possible to increase a carbon-based yield of propylene. An amount of water supplied to the reactor is preferably not less than 10 mol%, more preferably not less than 20 mol%, and further preferably not less than 30 mol%, relative to a total amount of 100 mol% of ethanol and acetone.

In the reaction step, it is preferable to gasify ethanol and acetone and supply the gasified ethanol and acetone to the reactor so that the gasified ethanol and acetone make contact with a catalyst. The catalyst only needs to be a catalyst for producing propylene from ethanol and acetone. For example, it is possible to use a catalyst that contains a component (A) which is zirconium oxide in an amount of not less than 50% by mass and less than 100% by mass, and a component (B) in an amount of more than 0% by mass and not more than 50% by mass (where a total mass of the component (A) and the component (B) is 100% by mass). Here, the component (B) is at least one element selected from the group consisting of lithium, sodium, potassium, rubidium, calcium, strontium, barium, scandium, yttrium, cerium, titanium, vanadium, chromium, copper, silver, gold, gallium, germanium, and tin.

A form of an element that is the component (B) may be a single entity of the element or may be an oxide of the element. If the element is in the form of an oxide, a mass of the component (B) is a mass of the element contained in the oxide, and a mass of oxygen is not included. The component (B) may be at least one element selected from the group consisting of lithium, sodium, potassium, rubidium, calcium, strontium, barium, scandium, yttrium, titanium, vanadium, chromium, copper, silver, gold, gallium, germanium, and tin.

The raw material may be brought into contact with the catalyst in the reactor, or the raw material may be brought into contact with the catalyst and then supplied to the reactor. The raw material may be in the form of liquid or gas. An example of a method for bringing the raw material into contact with the catalyst can be a method in which the raw material is gasified by heating, and the gasified raw material is supplied to a reactor that has been filled with the catalyst in advance.

The reactor may be any reactor that is capable of bringing the raw material into contact with the catalyst. Examples of the reactor include a fixed bed reactor, a fluidized bed reactor, a batch reactor, and the like. From the viewpoint of heat of reaction, the reactor may be an adiabatic reactor, an isothermal reactor, or a heat exchanging reactor.

A reaction temperature is preferably 270°C to 700°C, preferably 300°C to 650°C, and further preferably 350°C to 550°C.

A reaction pressure is preferably 10 kPa to 100000 kPa, and more preferably 100 kPa to 1000 kPa.

In view of stabilizing a gas composition supplied to the reaction step and stabilizing an operating condition of the reactor, the propylene production method according to an aspect of the present invention preferably further includes a dehydration step of separating water from the crude product obtained in the reaction step. In the dehydration step, water is removed by preferably not less than 10%, more preferably not less than 30%, relative to the number of moles of acetone contained in the fresh raw material.

(Gas-liquid separation step)

The gas-liquid separation step is a step of separating the crude product obtained in the reaction step into a gas phase containing propylene and a liquid phase containing acetone. The gas-liquid separation step is carried out in, for example, a gas-liquid separator illustrated in Fig. 1, and the crude product obtained in the reactor is supplied to the gas-liquid separator.

The gas-liquid separation step may be carried out by any method as long as a most part of propylene contained in the crude product can be separated into a gas phase and a most part of acetone contained in the crude product can be separated into a liquid phase. Therefore, the gas phase may contain a small amount of acetone. The liquid phase may also contain ethanol as well as acetone. In addition, the liquid phase may contain a low boiling point component (e.g., a dissolved gas component such as propylene, and acetaldehyde, etc.) having a boiling point lower than that of acetone.

A method of separating the crude product into a gas phase containing propylene and a liquid phase containing acetone is not particularly limited, and can be, for example, a method of carrying out separation by pressurization or cooling. When separation is carried out by cooling, a cooling temperature can be set, in accordance with pressure in the system, for example, to a temperature at which not less than 50% of acetone is present in the liquid phase and not less than 50% of propylene is present in the gas phase.

(Propylene recovery step)

The propylene recovery step is a step of recovering propylene (hereinafter referred to as product propylene) from the gas phase separated in the gas-liquid separation step. In the propylene recovery step, propylene may be recovered by any of the following (i) through (iii).

    (i) The gas phase is recovered directly as product propylene.
    (ii) The gas phase is pressurized to recover a condensed component containing propylene as product propylene.
    (iii) The condensed component obtained in (ii) is distilled to recover product propylene.

For example, (ii) is carried out with a compressor illustrated in Fig. 1, where the gas phase separated in the gas-liquid separator is supplied to the compressor. For example, (iii) is carried out in a propylene distillation column illustrated in Fig. 1. In this step, the condensed component obtained in (ii) is supplied to the propylene distillation column, and the condensed component is distilled in the propylene distillation column to be separated into product propylene and waste oil. From the viewpoint of increasing a degree of purification of propylene to be recovered, it is preferable to purify and recover propylene by at least one of the methods described in (ii) and (iii), and more preferably purify and recover propylene by the method described in (iii).

The pressure in (ii) is not particularly limited, as long that propylene is condensed at the pressure. If the pressure is low, electric power or the like taken for compression is small, and if the pressure is high, a condensation amount of propylene increases. Therefore, it is preferable to select an appropriate pressure for each device.

The distillation in (iii) is not particularly limited, and can be carried out with use of a propylene distillation technique in an existing naphtha cracker, propane dehydrogenation, a catalytic cracking process, and the like.

The condensed component may contain oxygenated hydrocarbons such as acetaldehyde and acetone. In the propylene recovery step, it is preferable that a condensed component containing oxygenated hydrocarbons such as acetaldehyde and acetone is further recovered. The recovered liquid phase is preferably supplied to at least one step selected from the group consisting of the reaction step, the gas-liquid separation step, and the recycling step. Thus, it is possible to increase a carbon-based yield of propylene by recycling not only oxygenated hydrocarbons such as acetaldehyde and acetone which are contained in the recycled raw material, but also oxygenated hydrocarbons such as acetaldehyde and acetone which are contained in the condensed component. In the propylene recovery step, a liquid phase containing oxygenated hydrocarbons such as acetaldehyde and acetone, which is a residue remaining after distillation of the condensed component obtained in (iii), is further recovered. The liquid phase may be supplied to at least one step selected from the group consisting of the reaction step, the gas-liquid separation step, and the recycling step.

The carbon-based yield of propylene obtained in the propylene recovery step is preferably 30 mol%, more preferably not less than 45 mol%, further preferably not less than 50 mol%, furthermore preferably not less than 55 mol%. An upper limit of the carbon-based yield of propylene obtained in the propylene recovery step is preferably as high as possible. For example, the upper limit can be not more than 92 mol%, not more than 85 mol%, not more than 80 mol%, or not more than 75 mol%.

Hydrogen ($H_2$) and carbon dioxide ($CO_2$) may each be recovered from the gas phase which is a residue remaining after pressurization in (ii). For example, a pressure swing adsorption (PSA) device may be used to separate the gas phase into hydrogen ($H_2$) and waste gas containing carbon dioxide ($CO_2$) to recover the hydrogen as a product.

The recovered propylene can be used as a raw material for polypropylene, propylene oxide, aromatic hydrocarbon, aromatic alcohol, and the like, and methods for producing these substances are also encompassed within the scope of the present invention.

(Recycling step)

The recycling step is a step of recovering a recycled raw material containing acetone from a liquid phase separated in the gas-liquid separation step. The recycled raw material recovered in the recycling step is supplied to the reaction step. A method of recovering a recycled raw material from a liquid phase can be, for example, a method in which the liquid phase is distillated to recover the recycled raw material. The recycled raw material may also contain oxygenated hydrocarbons such as ethanol, acetaldehyde, and isopropanol, and preferably contains ethanol and/or isopropanol. The recycling step is carried out, for example, in a recycled raw material distillation column illustrated in Fig. 1. In the recycling step, the liquid phase separated in the gas-liquid separator is supplied to the recycled raw material distillation column, the recycled raw material recovered in the recycled raw material distillation column is supplied to the reactor, and water is discharged from the recycled raw material distillation column. Thus, it is possible to increase a carbon-based yield of propylene by supplying the recycled raw material containing acetone to the reaction step for recycling.

A distillation condition for the recycled raw material distillation column is not particularly limited, and distillation in the recycled raw material distillation column can be carried out under any conditions.

A method of supplying the recycled raw material to the reaction step is not particularly limited. The recycled raw material may be mixed with a fresh raw material in advance and supplied to the reactor. Alternatively, the fresh raw material and the recycled raw material may be separately supplied to the reactor.

The propylene production method according to an aspect of the present invention preferably further includes the steps of: recovering, from the liquid phase, a low boiling point component having a boiling point lower than that of acetone; and supplying the low boiling point component to at least one step selected from the group consisting of the reaction step, the gas-liquid separation step, and the recycling step. The low boiling point component includes, for example, acetaldehyde and the like. Thus, it is possible to increase a carbon-based yield of propylene by recycling not only the recycled raw material containing acetone but also the low boiling point component.

The present invention is not limited to the embodiments, but can be altered variously by a skilled person in the art within the scope of the claims. The present invention also encompasses, in its technical scope, any embodiment derived by appropriately combining technical means disclosed in differing embodiments.

Examples

[Simulation Example 1]

A propylene production method including the following steps (1) through (4) was simulated to calculate a theoretical value of a yield.

(1) Reaction step

As fresh raw materials, liquid ethanol and liquid acetone are gasified and supplied to a reactor. Here, an amount of the liquid acetone is 0.5 moles per mole of the liquid ethanol. Further, as a recycled raw material, acetone is gasified and supplied to the reactor in an amount of 0.7 moles per mole of the liquid ethanol. The reactor used is a reactor filled with a catalyst containing 1 part by mass of a component (A) which is zirconium oxide and 0.009 parts by mass of a component (B) which is calcium. The ethanol and acetone supplied to the reactor are brought into contact with the catalyst.

(2) Gas-liquid separation step

A crude product recovered from the reactor outlet is cooled, and a high boiling point component is condensed and separated into a liquid phase containing acetone and a gas phase containing propylene.

(3) Propylene recovery step

The gas phase separated in the gas-liquid separation step is extracted, and propylene is recovered.

(4) Recycling step

A portion of water is separated from the liquid phase separated in the gas-liquid separation step, and the recycled raw material containing 0.7 moles of acetone per mole of ethanol in the fresh raw material is supplied to the reactor.

(Calculation of theoretical value of yield)

A carbon-based propylene concentration in the crude product at the reactor outlet in a steady state was assumed to be 35%.

A theoretical value of a yield was calculated according to formula (I) below based on: a molar ratio between ethanol and acetone in the fresh raw material assumed as described above; a molar ratio of acetone in the recycled raw material to ethanol in the fresh raw material; and a propylene concentration in the crude product.

$$\text{Formula (I): } Y = X/100 \times (Fe+Fa+Ra)/(Fe+Fa) \times 100$$

Y: Yield of propylene
X: Carbon-based propylene concentration in crude product at reactor outlet
Fe: Number of moles of carbon in ethanol contained in fresh raw material
Fa: Number of moles of carbon in acetone contained in fresh raw material
Ra: Number of moles of carbon in acetone as oxygenated hydrocarbon contained in recycled raw material

Formula (I) indicates a theoretical value of a yield on the premise that there is no loss of the fresh raw material and the recycled raw material. That is, the yield was calculated for a case in which the number of moles of all carbons supplied to the reactor (Fe+Fa+Ra) matches the number of moles of all carbons at the reactor outlet.

When the theoretical value of the yield was calculated, it was assumed to be 56%, as shown in formula (II) below.

$$\text{Formula (II): } 56 = 0.35 \times \{1 \times 2 + (0.7+0.5) \times 3\}/(1 \times 2 + 0.5 \times 3) \times 100$$

[Comparative Simulation Example 1]

The carbon-based propylene concentration in the crude product at the reactor outlet is 35% if step (1) of Simulation Example 1 above was included, steps (2) through (4) were not included, and 1.2 moles of liquid acetone was supplied as a fresh raw material per mole of liquid ethanol in step (1). That is, the theoretical value of the yield was assumed to be 35%.

[Preparation Example 1]

Zirconium oxide (RC-100, manufactured by DAIICHI KIGENSO KAGAKU KOGYO CO., LTD.) was sized to 0.3 mm to 0.6 mm. To 5.00 g of the sized zirconium oxide, 1.20 g of a solution constituted by 0.0889 g of calcium acetate monohydrate and 1.11 g of millipore water was added and permeated in small amounts to obtain a pore filling body. This pore filling body was maintained in air at 120°C for 3 hours and then in air at 500°C for 2 hours to obtain a catalyst A.

[Example 1 (one-pass experiment)]

A quartz reaction tube was filled with 1.25 g of the catalyst A. A mixture gas having a molar ratio of ethanol/acetone/water/nitrogen = 8/4/74/14 was supplied to the reaction tube at a rate of 96 ml/min, and reaction was carried out under conditions of 0.2 MPa-G and 450°C. A gas and a fluid discharged from the gas outlet of the reaction tube were collected 1.5 hours later from initiation of the reaction, and analyzed by gas chromatography to determine a product composition. The product composition was expressed by (number of moles of carbon in product) / (number of moles of carbon in raw material compound subjected to reaction) $\times$ 100 (%). In the product composition, propylene was 26.1%, ethylene was 11.5%, $CO_2$ was 9.3%, acetone was 29.0%, and IPA was 1.8%. When another component appearing as a peak other than the above compound on gas chromatography was assumed to be 1-pentene, a product composition of the another component was 1.5%.

[Example 1 (simulation)]

The following conditions were set, and then a material balance with recycling and a material balance without recycling were obtained by process simulation.

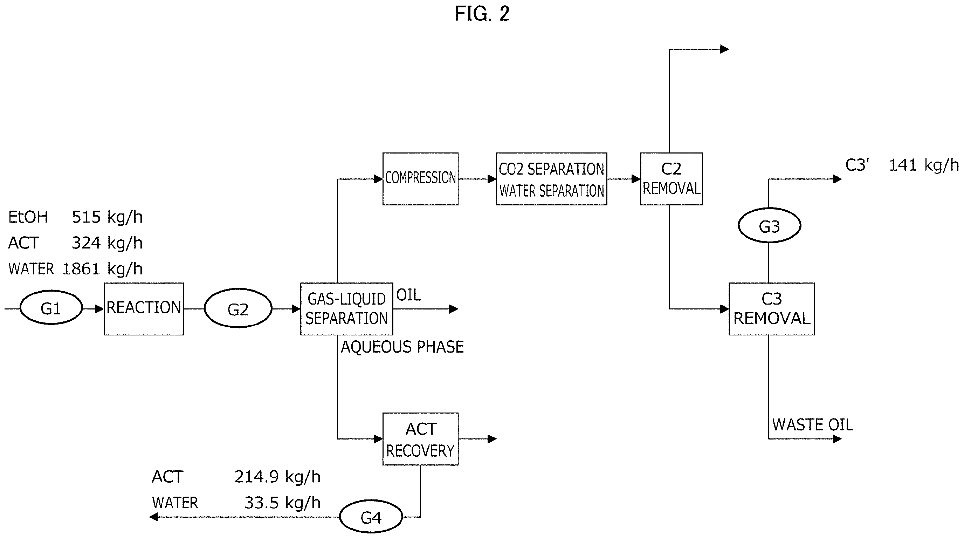

- As illustrated in Fig. 2, the process flow included a reaction step, a gas-liquid separation step, an acetone (ACT) recovery step, a compression step, a $CO_2$ separation-water separation step, a C2 removal step, and C3 removal step.
- The acetone recovery step (recycled raw material recovery step) was flash distillation of an aqueous phase containing acetone.
- As the material balance at an outlet of the reaction step, a material balance calculated from the result of the gas chromatography in Example 1 was referred to. A material balance of a carbon component was adjusted while assuming that hexane was generated.
- For a hydrogen balance and an oxygen balance, it was assumed that water and hydrogen were generated.

The simulation results are shown in Table 1. The results at the reactor inlet G1 and the reactor outlet G2 were made to conform to the material balance of the supplied raw materials and the material balance at the reactor outlet in the one-pass experiment results. Results at G3 after the C3 removal step and at G4 after acetone recovery (after recycled raw material recovery) are simulation results.

As a result of the simulation, it has been found that, in the steady state, acetone in the fresh raw material can be reduced by returning, to the reaction step, the recovered recycled portion of an aqueous acetone solution which has undergone gas-liquid separation. With acetone recycling, the propylene yield is 35.9%.

[Comparative Example 1]

As a result of the simulation, it has been found that, when a recovered portion of an aqueous acetone solution which has undergone gas-liquid separation is not recycled, acetone in the fresh raw material cannot be reduced. Without acetone recycling, the propylene yield is 25.7%.

[Table 1]

| (kg/h) | G1 | G2 | G3 | G4 |
|---|---|---|---|---|
| Ethanol | 514.52 | 10.29 | 0.00 | 9.26 |
| Propylene | 0.00 | 141.47 | 141.11 | 0.36 |
| Ethylene | 0.00 | 62.33 | 0.00 | 0.11 |
| Methane | 0.00 | 3.72 | 0.00 | 0.00 |
| Carbon dioxide | 0.00 | 158.16 | 0.00 | 1.91 |
| Acetone | 324.33 | 216.96 | 0.00 | 214.93 |
| Isopropanol | 0.00 | 13.93 | 0.00 | 13.93 |
| 1-pentene | 0.00 | 20.06 | 1.08 | 0.39 |
| Hexane | 0.00 | 99.90 | 0.00 | 0.39 |
| H2 | 0.00 | 15.20 | 0.00 | 0.01 |
| H2O | 1861.14 | 1957.96 | 0.00 | 33.46 |

Industrial Applicability

The present invention can be utilized in a method for producing propylene.

**Claims**

1. A propylene production method, comprising:

   a reaction step of supplying a fresh raw material containing ethanol and acetone and a recycled raw material containing acetone to a reactor so that the fresh raw material and the recycled raw material react to obtain a crude product;
   a gas-liquid separation step of separating the crude product into a gas phase containing propylene and a liquid phase containing acetone;
   a propylene recovery step of recovering propylene from the gas phase; and
   a recycling step of recovering the recycled raw material from the liquid phase.

2. The propylene production method according to claim 1, wherein:
   in the reaction step, a molar ratio of acetone contained in the fresh raw material supplied to the reactor to ethanol contained in the fresh raw material is 0.010 to 1.7.

3. The propylene production method according to claim 1 or 2, further comprising the steps of:

   recovering, from the liquid phase, a low boiling point component having a boiling point lower than that of acetone; and
   supplying the low boiling point component to at least one step selected from the group consisting of the reaction step, the gas-liquid separation step, and the recycling step.

4. The propylene production method according to claim 1 or 2, further comprising the steps of:

   further recovering a condensed component containing acetone in the propylene recovery step; and
   supplying the condensed component to at least one step selected from the group consisting of the reaction step, the gas-liquid separation step, and the recycling step.

5. The propylene production method according to any one of claims 1 through 4, wherein:
   the recycled raw material contains ethanol and/or isopropanol.

6. The propylene production method according to any one of claims 1 through 5, further comprising:
   a dehydration step of separating water from the crude product which has been obtained in the reaction step.

9

**7.** The propylene production method according to any one of claims 1 through 6, wherein:
in the reaction step, water is also supplied to the reactor.

**8.** The propylene production method according to claim 7, wherein:
an amount of the water supplied to the reactor in the reaction step is not less than 10 mol%, relative to a total amount of 100 mol% of ethanol and acetone which are supplied to the reactor.

FIG. 1

WASTE GAS $CO_2$

PSA DEVICE ——→ PRODUCT $H_2$

COMPRESSOR

PROPYLENE DISTILLATION COLUMN

→ PRODUCT PROPYLENE

FRESH RAW MATERIAL

RECYCLED RAW MATERIAL

→ WASTE OIL

REACTOR

GAS-LIQUID SEPARATOR

RECYCLED RAW MATERIAL DISTILLATION COLUMN

→ DRAINAGE

FIG. 2

EtOH 515 kg/h
ACT 324 kg/h
WATER 1861 kg/h

COMPRESSION

CO2 SEPARATION
WATER SEPARATION

C2 REMOVAL

C3' 141 kg/h

G3

G1 → REACTION → G2 → GAS-LIQUID SEPARATION → OIL

AQUEOUS PHASE

C3 REMOVAL

WASTE OIL

ACT RECOVERY

ACT 214.9 kg/h
WATER 33.5 kg/h

G4

EP 4 289 806 A1

# EP 4 289 806 A1

## INTERNATIONAL SEARCH REPORT

International application No.

**PCT/JP2022/002835**

### A. CLASSIFICATION OF SUBJECT MATTER

*C07C 1/20*(2006.01)i; *C07C 11/06*(2006.01)i
FI: C07C1/20; C07C11/06

According to International Patent Classification (IPC) or to both national classification and IPC

### B. FIELDS SEARCHED

Minimum documentation searched (classification system followed by classification symbols)

C07C1/20; C07C11/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JST7580/JSTChina (JDreamIII)

### C. DOCUMENTS CONSIDERED TO BE RELEVANT

| Category* | Citation of document, with indication, where appropriate, of the relevant passages | Relevant to claim No. |
|---|---|---|
| Y | JP 2016-150932 A (IDEMITSU KOSAN CO., LTD.) 22 August 2016 (2016-08-22) claims 1-9, paragraphs [0021]-[0029], fig. 1 | 1-2, 6-8 |
| A | | 3-5 |
| Y | JP 2012-254447 A (TOKYO INST. OF TECHNOLOGY) 27 December 2012 (2012-12-27) claims 1, 5, paragraphs [0028], [0035], [0036], [0088], table 3, paragraphs [0094]-[0102], table 4 | 1-2, 6-8 |
| A | | 3-5 |
| A | JP 2013-252495 A (SUMITOMO CHEMICAL CO., LTD.) 19 December 2013 (2013-12-19) entire text | 1-8 |
| A | JP 2012-240914 A (TOKYO INST. OF TECHNOLOGY) 10 December 2012 (2012-12-10) entire text | 1-8 |

☐ Further documents are listed in the continuation of Box C.    ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>**14 March 2022** | Date of mailing of the international search report<br><br>**22 March 2022** |
| Name and mailing address of the ISA/JP<br><br>**Japan Patent Office (ISA/JP)**<br>**3-4-3 Kasumigaseki, Chiyoda-ku, Tokyo 100-8915**<br>**Japan** | Authorized officer<br><br><br><br>Telephone No. |

Form PCT/ISA/210 (second sheet) (January 2015)

## INTERNATIONAL SEARCH REPORT
### Information on patent family members

International application No.

**PCT/JP2022/002835**

| Patent document cited in search report | | | Publication date (day/month/year) | Patent family member(s) | Publication date (day/month/year) |
|---|---|---|---|---|---|
| JP | 2016-150932 | A | 22 August 2016 | (Family: none) | |
| JP | 2012-254447 | A | 27 December 2012 | WO 2012/157578 A1 claims 1, 5, paragraphs [0028], [0035], [0036], [0088], table 3, paragraphs [0094]-[0102], table 4 | |
| JP | 2013-252495 | A | 19 December 2013 | (Family: none) | |
| JP | 2012-240914 | A | 10 December 2012 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **IWAMOTO, M. et al.** Pulse and IR study on the reaction pathways for the conversion of ethanol to propene over scandium-loaded indium oxide catalysts. *ACS Catal.,* 2014, vol. 4 (10), 3463-3469